# EUROPÄISCHE PATENTANMELDUNG

(11) **EP 1 219 170 A2**
(43) Veröffentlichungstag der Anmeldung: **03.07.2002**
(21) Anmeldenummer: 01130645.3
(22) Anmeldetag: 21.12.2001
(51) Int. Cl.: A01K 29/00, A01K 1/03

(54) **Verfahren und Vorrichtung zur Dokumentation des Gesundheits- und Krankheitszustandes grosser Nutztiere**

(30) Priorität: 29.12.2000 DE 10065700; 23.01.2001 DE 10102955
(71) Anmelder: Voit, Stefan, Dipl.-Wirtsch.-Ing., 66386 St. Ingbert (DE)
(72) Erfinder: Foss, Pierre Nicholas, Dr. med., 66687 Wadern (DE)
(74) Vertreter: Vièl, Christof, Dipl.-Ing.

(57) **Zusammenfassung**

Die Erfindung betrifft ein Verfahren und eine Vorrichtung zur Dokumentation des Gesundheits- und Krankheitszustandes großer Nutztiere. Unter großen Nutztieren werden Schweine, Rinder, Pferde, Kamele und dergleichen verstanden.

Um zur sachgemäßen Überprüfung und Dokumentierung des Zustandes großer Nutztiere ein schnelles, möglichst genaues, nicht invasives Prüfverfahren zu schaffen, die eine sofortige Dokumentation des Zustandes zahlreicher Tiere innerhalb kurzer Zeit erlaubt, wird im Rahmen der Erfindung vorgeschlagen, dass jedes Nutztier in eine standardisierte, definierte Prüfumgebung eingebracht wird, in der rechnergesteuert, halb- oder vollautomatisiert und nicht invasiv die optisch-apparativ sichtbaren und die neurophysiologischen-neuromuskulären Zustandsbilder und Reaktions- und Verhaltensweisen des Tieres auf standardisierte, definierte, rechnergestützte akustische, optische, mechanische, taktile, neurophysiologische oder elektrische Provokationsreize aufgezeichnet und gespeichert werden.

## Beschreibung

Die Erfindung betrifft ein Verfahren und eine Vorrichtung zur Dokumentation des Gesundheits- und Krankheitszustandes großer Nutztiere.

Unter großen Nutztieren werden Schweine, Rinder, Pferde, Kamele und dergleichen verstanden.

Die Bovine Spongiforme Enzephalopathie (BSE, Rinderwahnsinn) wurde erstmals 1984 in England von einem Tierarzt festgestellt. Allein in England sind durch Verfütterung von Tiermehl an die sich vegetarisch ernährenden Kühe mehr als 160 000 Kühe erkrankt. Über 3 Millionen Kühe wurden geschlachtet. Der Landwirt, der ein sogenanntes ZNS (**Z**entrales **N**erven **S**ystem)-auffälliges Tier in seinem Stall entdeckt, muss dies melden. Er soll generell 2-3 mal im Jahr das Rind auf Krankheiten untersuchen. Prof. Ueli Braun von der Klinik für Wiederkäuer- und Pferdemedizin der Universität Zürich hat in seiner Veröffentlichung "Klinische Befunde, diagnostisches Vorgehen und Differentialdiagnosen bei BSE" die einzelnen Störungen der BSE, die beim Rind auftreten genau aufgezählt.

Prof. Braun hat auch ein Aufklärungsvideo über die Reaktionsweisen BSE-erkrankter Rinder herausgegeben. Die BSE ist eine Krankheit, die durch Störungen des Verhaltens, der Sensibilität und der Bewegung charakterisiert ist. Die von Prof. Braun vorgeschlagenen einfachen Testvorschläge der Reaktionsweisen sind jedoch in ihrer beschriebenen Ausführung nicht standardisierbar und lassen keine exakten und vergleichbaren Analysen und Dokumentationen zu. Auch die Epidemie der Maul- und Klauenseuche in diesem Jahr kostete viele Tiere das Leben und führte ebenfalls wie die BSE zu milliardenschweren Schäden der Landwirtschaft und zu erheblichen Umsatzeinbußen im Vertrieb des Fleischhandels. Der Verbraucher legt seither deutlich mehr Wert auf die Herkunft und Qualität des Fleisches.

Wie schon erwähnt ist der Landwirt angehalten, seine Rinder 2-3 mal in Jahr zu untersuchen und dies. zu protokollieren. Es existieren jedoch Tierherden, die mehrere hundert bzw. mehrere tausend Rinder zählen, so dass der Landwirt nicht jedes Tier kennen und seine Aufzucht beobachtet kann. Auch wissen die Schlachtereien und die Molkereien nicht, ob der Landwirt seine Tiere richtig betreut. In den Schlachtereien wird eine recht oberflächliche Tierbeschau vor der Schlachtung durch den Tierarzt durchgeführt. Da in den großen Schlachtereien am Tag mehrere tausend Tiere getötet werden, kann es sich bei dieser Tierbeschau nur um eine schnelle und oberflächliche Prüfung handeln, die unter den Augen des Tierarztes abläuft. Dabei werden keine optischen dokumentativen oder diagnostischen Hilfsmittel verwendet oder neurophysiologische apparative Untersuchungen durchgeführt.

Es stellt sich daher die Aufgabe, zur sachgemäßen Überprüfung und Dokumentierung des Zustandes großer Nutztiere ein schnelles, möglichst genaues, nicht invasives Prüfverfahren und eine entsprechende -anlage zu schaffen, die eine sofortige Dokumentation des Zustandes zahlreicher Tiere innerhalb kurzer Zeit erlaubt. Eine Prüfanlage also, die eine zweckmäßige Überwachung großer Rinderherden oder großer Schlachttierzahlen garantiert, ohne den Betriebsfluss zu stören und die die gewünschten Qualitätskriterien der Betreiber einhält.

Die Erfindung löst diese Aufgabe dadurch, dass jedes Nutztier in eine standardisierte, definierte Prüfumgebung eingebracht wird, in der rechnergesteuert, halb- oder vollautomatisiert und nicht invasiv die optisch-apparativ sichtbaren und die neurophysiologischen-neuromuskulären Zustandsbilder und Reaktions- und Verhaltensweisen des Tieres auf standardisierte, definierte, rechnergestützte akustische, optische, mechanische, taktile, neurophysiologische oder elektrische Provokationsreize aufgezeichnet und gespeichert werden.

Indem die Tiere in eine definierte, standardisierte Prüfumgebung gebracht werden und dort vollautomatisch rechnergesteuert mit optischen und neurophysiologischen Methoden und Apparaten analysiert werden, können diese Tiere faktisch im Durchgehen durch die Prüfumgebung sehr schnell gecheckt und deren Reaktionen dokumentiert werden.

Auf diese Weise können in kurzer Zeit viele Nutztiere automatisiert, objektiv und vergleichbar auf ihren Gesundheitszustand oder mögliche Krankheitsbilder überprüft werden, und eine automatisierbare Früherkennung von Krankheiten wird möglich.

Eine Weiterbildung der Erfindung besteht darin, dass die Zustandsbilder und Reaktions- und Verhaltensweisen analysiert und - vorzugsweise sofort - bewertet werden.

Mit Hilfe des erfindungsgemäßen Verfahrens kann somit der Stall, die Schlachterei oder die Molkerei gleichzeitig zu einem kostengünstigen, hochwertigen Analyse- und Dokumentationszentrum des Gesundheitszustandes der Nutztiere gemacht werden, wobei diese schnell, aussagekräftig und kompetent mit neuester apparativer Technik überprüft werden. Der Tiererzeuger, der Lebensmittelverarbeiter, der Vertreiber und der Verbraucher können auf ein exaktes Dokument des verarbeitenden Nutztieres zurückgreifen.

Erfindungsgemäß ist vorgesehen, dass als Prüfumgebung festgelegte Prüfstrecken in Tierställen oder Schlachthäusern, festgelegte Prüfräume in Tierställen, Prüfboxen, Prüfzimmer oder überdachte Prüfunterstände bei Freilandhaltung genutzt werden.

Diese Prüfstrecken sind insbesondere dann sinnvoll, wenn an einem Ort regelmäßig eine größere Anzahl an Nutztieren zu prüfen ist, insbesondere in größeren Betrieben.

Werden die Prüfumgebungen als fahrbare Analyseboxen eingesetzt, die in Aufbauten von LKW eingebaut werden können, so ist es möglich, die Box zu jedem Landwirt zu fahren und dessen Tiere überprüfen zu lassen.

Eine Ausbildung der Erfindung besteht darin, daß die Tierprüfungen durch Bewegungsmelder oder Kontakte automatisch eingeschaltet werden.

Weiterhin kann vorgesehen sein, daß die Bewertungen und Daten der Prüfungen zu Tierärzten und Tierkliniken übertragen und von diesen spezielle Prüfprogramme angesteuert werden können.

Es liegt im Rahmen der Erfindung, dass die in den Prüfumgebungen eingesetzten Tierarretierungen und/oder in den Tierarretierungen Gestängeelemente bewegt, rotiert oder gekippt werden, um das Tier zu definierten Bewegungen zu zwingen oder taktil zu reizen.

Ebenso ist es möglich, dass zumindest Teilbereiche des Bodens der Prüfvorrichtung bewegt werden.

Diese beiden Vorgehensweisen dienen der Ausübung eines Reizes auf das Nutztier, woraufhin die Reaktion des Nutztieres dokumentiert werden kann.

Eine bevorzugte Ausführungsform der Erfindung besteht darin, dass mehrere Prüfumgebungen, insbesondere in Form von Prüfboxen, nebeneinander oder hintereinander in Form einer Prüfreihe oder Prüfkette genutzt werden und jede Prüfumgebung bzw. Prüfbox verschiedene Prüfschwerpunkte ausführt.

Durch die verschiedenen Ausprägungen der Prüfumgebungen und den modulartigen, apparativen Aufbau des Instrumentariums können diese kundenspezifisch genau auf die Belange des Anwenders zugeschnitten werden.

Durch die Möglichkeit die Prüfumgebungen aneinander zu reihen, also eine Prüfkette aufzubauen, können eine große Anzahl der Nutztiere faktisch am Fließband im Durchlaufen analysiert werden. Solche Prüfketten würden sich für große Schlachtereien eignen, die auffällige oder kranke Tiere sofort, direkt vor der Schlachtung, aussortieren. Der Schlachthof hat dann ein exzellentes Dokumentationsmittel zur Hand, um die Qualität seiner Schlachttiere nach neustem und höchstem Niveau forensisch sicher zu belegen.

Ausbildungen der Erfindung bestehen darin, dass zusätzlich telethermographische Prüfungen, Woodlichtanalysen, Röntgen- und Ultraschalluntersuchungen, dreidimensionale Vermessungen der Tiere, Gewichts- und Größenmessungen oder Prüfungen der Gesichtsmimik bzw. der Augenbewegungen der Tiere erfolgen.

Es ist besonders vorteilhaft, dass die Tiere im Verlaufe des Lebens mehrfach untersucht werden und deren Bewertung miteinander und mit den Bewertungen der jeweiligen Herde verglichen werden.

Durch Verlaufskontrollen der Prüfungen eines Tieres im Verlaufe seines Lebens können gleichzeitig exakte, forensisch sichere Dokumentation des Gesundheitszustandes der Tiere gefertigt werden. Der Landwirt kann mit Hilfe der Erfindung schnell und kostengünstig einen exakten Überblick über den Gesundheitszustand seiner Tiere gewinnen und auffällige Tiere früh von den gesunden Tieren unterscheiden.

Im Rahmen der Erfindung liegt auch eine Vorrichtung zur Dokumentation des Gesundheitsund Krankheitszustandes großer Nutztiere, wobei eine Prüfvorrichtung vorgesehen ist, die Mittel zum Ausüben von akustischen, optischen, mechanischen, taktilen, neurophysiologischen oder elektrischen Provokationsreizen aufweist, und daß Mittel zum Aufzeichnen und Speichern der dadurch hervorgerufenen Zustandsbilder und Reaktions- und Verhaltensweisen des Tieres vorgesehen sind.

Hierbei ist es zweckmäßig, dass Mittel zum Analysieren und Bewerten der Zustandsbilder und Reaktions- und Verhaltensweisen des Tieres vorgesehen sind.

Die umgehende Auswertung der Information ermöglicht eine schnelle Entscheidung, ob das untersuchte Tier den Gesundheitskriterien genügt oder ob weitere Untersuchungen erforderlich sind.

Eine Ausgestaltung der Erfindung sieht vor, dass Mittel zum Bewegen, Rotieren oder Kippen der als Prüfumgebung eingesetzten Tierarretierungen und/oder Gestängeelementen in den Tierarretierungen vorgesehen sind.

Weiterhin kann es vorteilhaft sein, dass zumindest Teilbereiche des Bodens der Prüfvorrichtung bewegbar sind.

Dies kann beispielsweise durch einzeln anblasbare Luftkammern im Bodenbereich erfolgen.

Ebenso liegt es im Rahmen der Erfindung, dass zur neurophysiologischen und neuromuskulären Prüfung spezielle Elektroden in Bürsten oder Krallenformen mit Bandhalterungen oder an dreidimensional steuerbaren Kontaktarmen oder mit Elektrodenvergurtungen für den Kopfsitz vorgesehen sind.

Darüber hinaus kann es sinnvoll sein, dass zusätzlich Mittel für telethermographische Prüfungen, Woodlichtanalysen, Röntgen- und Ultraschalluntersuchungen, dreidimensionale Vermessungen der Tiere, Gewichts- und Größenmessungen oder Prüfungen der Gesichtsmimik bzw. der Augenbewegungen der Tiere vorgesehen sind.

Es kann zweckmäßig sein, dass mindestens eine Prüfvorrichtung auf einer Fördervorrichtung für den Transport der Nutztiere angeordnet ist.

Dies ermöglicht es, während des Transportes der Nutztiere diese zu untersuchen.

Weiterhin ist es möglich, dass mehrere Prüfvorrichtungen sternförmig angeordnet sind.

Hierdurch wird eine platzsparende Untersuchung der Tiere möglich.

Weiterhin liegt es im Rahmen der Erfindung, dass die Prüfvorrichtung transportierbar ist.

Dies ermöglicht es beispielsweise Tierärzten, die Prüfvorrichtung mitzuführen und vor Ort aufzubauen.

### Die Untersuchungen werden nun praktisch erläutert:

Die Rinder des Landwirtes kommen abends von selbst in den Stall zurück. Sie könnten am Hals ein Band mit einem Sender tragen, der zur Identifizierung des Rindes dient. Die Tiere betreten einzeln die Stallgasse, um in ihre Ruhe- oder Futterboxen zu gelangen. Hierbei können die Bewegungen der Tiere schon geprüft werden. Die Tiere werden nun durch definierte Lichtblitze oder Knalllaute gereizt und videografisch beobachtet. Die Tiere, die auffällig reagieren z.B. durch Erschrecken, Fallen, Angst usw., werden registriert.

Während des Schlafens der Tiere können über Bewegungsmelder oder Infrarotkameras die Schlafruhe oder das Aggressionsverhalten der Tiere registriert werden.

Das Bewegungsmuster der Tiere kann definiert überprüft werden, indem in den Ein- oder Ausgängen der Ställe die Bodenebenen gezielt verändert werden oder im Weg kleinere Hindernisse eingebaut werden.

Mit Hilfe solcher einfacher Testungen, die voll automatisiert sind, wenig Zeit und Raum und keine menschliche Überwachung bedürfen, können die Tiere sehr genau auf subklinische Störungen überprüft werden. Die Erkrankungen des zentralen Nervensystems können hierdurch wesentlich früher erkannt werden als dies bisher dem Landwirt möglich war. Dies vor allem dadurch, weil bei jedem Tier auffällige Werte mit früheren Werten des gleichen Tests verglichen werden können und deshalb die individuellen und dann evtl. auch krankhaften Unterschiede in den Provokationsreaktionen exakt herausgefunden werden können.

Die Testreaktionen können somit statistisch und individuell geeicht werden, was eine sehr gute Erkennungsgenauigkeit verbürgen würde.

Der Landwirt selbst braucht die Analyse nicht zu überwachen. Die Testreaktionen werden automatisch über eine Software registriert und analysiert. Der Landwirt braucht nur die Ergebnisse zu betrachten, die er jederzeit z.B. am folgenden Morgen abrufen kann. Fällt ein Rind bei der Basisprüfung durch krankhafte oder suspekte Testreaktionen auf, so wird es über ein spezielleres Analyseprogramm, evtl. an einem anderen Tag, weitergehend überprüft. Bestätigt sich danach weiter der Verdacht auf eine Störung, so kann der Tierarzt hinzugezogen werden oder es werden die Ergebnisse der Testungen über elektronische Medien an Spezialisten übermittelt. Diese Spezialisten, die z.B. in den tierärztlichen Hochschulen oder in den Ministerien der Landwirtschaft zur Verfügung ständen, könnten durch direkten Zugriff auf die Software der Analyse noch spezifischere Testungen veranlassen.

Durch solche Kontrollen und Hilfestellungen würde der Landwirt in seinem Bestreben sehr unterstützt werden, einen wirklich gesunden Tierbestand aufzuziehen und Krankheiten schon im Beginn zu erkennen. Außerdem wäre exakt zu überprüfen, ob alle Tiere des Bestandes auch analysiert wurden.

Die optische Überwachung der Tiere kann auch so weit gehen, dass sie mit optischen Vergleichsanalysen oder Erkennungsanalysen ergänzt würden. Diese Analysen würden z.B. das einzelne Tier automatisch identifizieren, deren Fellfarbstrukturen oder deren Gangbild vergleichen und bei Veränderungen Alarm geben. So können z.B. Pilzerkrankungen des Felles sehr früh erkannt werden. Diese Pilzdiagnostik wäre noch durch die Verwendung robuster und kostengünstiger Fluoreszenzstrahler zu verbessern, deren Strahlen im pilzbefallenen Fell der Rinder typische Lichterscheinungen auslösen würden, die kleinste Pilznester zur Entdeckung bringen würden.

Weitere optische Analysenhilfen könnten Infrarotkameras sein. Das aufgenommene Wärmebild des Tieres könnte exakt und frühzeitig Durchblutungsstörungen und vegetativneuronale Schäden aufzeigen.

Diese Kameras könnten auch zur nächtlichen Überwachung des Stalles eingesetzt werden und aggressive oder hyperaktive Tiere detektieren, die Verhaltensauffälligkeiten zeigen.

Der Landwirt und die verantwortungsvolle Landwirtschaft haben hier erstmals ein Verfahren und ein Geräteinstrumentarium zur Verfügung, das den hohen, in Zukunft erforderlichen Analyse- und Dokumentationsstandard mit kostengünstigen Erwerb und neuesten technischen Mitteln vereint.

Jedem Tier einer Herde könnte eine Dokumentationsspeichermedium zugeteilt werden, auf dem sämtliche Ergebnisse und digitalisierte Bilder des Tieres abgespeichert sind und das beim Verkauf des Tieres weitergegeben wird.

Nachfolgend wird die Erfindung anhand eines bevorzugten Ausführungsbeispieles einer sehr komfortablen Ausstattung der Prüfumgebung zur Tierprüfung beschrieben.

In einem großen Schlachthof, in dem täglich über tausend Rinder geschlachtet werden, stehen in einer Reihe nebeneinander 5 Prüfboxen gleicher Ausstattung. Der Betreiber des Schlachthofs lässt jedes Tier vor der Schlachtung auf erfindungsgemäße Weise dokumentieren und vollautomatisch gegen Vergleichswerte abchecken. Er kann so den Zustand der Rinder objektiv und standardisiert für seine Qualitätssicherung dokumentieren und gleichzeitig auch die Tiere auf Krankheitssymptome optisch und nicht invasiv überprüfen. Erkrankte oder auffällige Tiere, die den Tier-Test nicht bestehen, werden für die Schlachtung nicht zugelassen und dem Tiererzeuger zurückgegeben bzw. werden die Tiere dann noch genauer tierärztlich überprüft. Der Schlachthofbetreiber kann sich hierdurch weitgehend vor Regressforderungen und infektionsbedingten Störungen seines Betriebes schützen.

Er kann die Tiererzeuger hochtechnisch anhand des Tierproduktes überwachen. Er gewinnt Gütesicherheit und Qualitätsdokumente seiner Produkte schon zu Beginn der Verarbeitungskette, und zwar ohne invasive Eingriffe, ohne die Tiere zu schädigen oder zu quälen und kann wegen der Standardisierung der Prüfungen die Ergebnisse miteinander vergleichen.

Die Tiere werden direkt nach der Ausladung im Schlachthof in Reihen hintereinander vor den Boxen positioniert. Automatisch öffnen sich die Boxentüren und je ein Rind betritt die Tierarretierung, die mitten in einem mit definiertem Licht ausgeleuchteten Raum steht. Die Boxentüren schließen sich wieder und das Tier steht von allen Seiten von stabilen Gittern begrenzt zur Prüfung bereit. Das Tier wird nun zuerst einmal digital videografisch in Farbe mit mehreren, festinstallierten, fernsteuerbaren Kameras zur Erkennung und Dokumentierung des äußeren Zustandes des Tieres aufgenommen. In der Arretierung wird es dann gewogen, seine Größe und Kontur festgestellt über Referenzmaßstäbe, die an der Arretierung oder den Wänden der Prüfumgebung befestigt oder markiert sind. Nun wird das Raumlicht abgedunkelt und das Woodlicht eingeschaltet. Dieses Woodlicht dient zur Fluoreszenzanregung, um damit Pilzerkrankungen im Fell des Tieres zum Leuchten zu bringen. Hiervon wird ebenfalls ein digitales Videobild gemacht. Danach werden im gedämpften Licht standardisierte Lichtblitze von einem speziellen Blitzgerät abgegeben und die Reaktionen bzw. Verhaltensweisen des Tieres auf diese optischen Reize videografisch dokumentiert.

Entsprechend werden auch die äußeren Reaktionen des Tieres auf standardisierte Knalllaute dokumentiert. Nun wird das Wärmebild des Tieres bei den standardisierten Raumbedingungen (Lichtverhältnisse, Raumtemperaturen bekannt und konstant) über Infrarotkameras aufgezeichnet. Hierdurch sind generelle Temperaturerhöhungen, lokale Entzündungen an Gelenken, der Lunge, der Nasennebenhöhlen, den Hufen oder Trächtigkeiten usw. sofort zu erkennen. Dann werden vollautomatisch gesteuert am Arretierungsgestänge mehrere gepolsterte Metallarme in die Tritt- bzw. Stehfläche des Tiere eingefahren, die das Tier dazu zwingen bestimmte Bewegungen wie z.B. Anheben der Beine bzw. Übersteigen der Metallarme zu vollziehen.

Außerdem können über diese Metallarme dem Tier milde Stromstöße appliziert werden, die die Schmerzempfindlichkeit des Tieres prüfen, was durch Zucken, Anheben oder Wegziehen der Gliedmaßen beantwortet und wiederum digital videographiert wird. Sind diese Prüfungen ohne pathologische Erkenntnisse ausgegangen, wird automatisch die Tierarretierung auf der anderen Raumseite geöffnet, ebenso die Raumtüre und das Rind zur Schlachtung freigegeben. Anderenfalls wird das Rind danach ebenfalls automatisch ausgesondert, evtl. einem Veterinär zur genauen Inspektion vorgestellt oder auch dem Tiererzeuger oder Mäster zurückgegeben.

Der Schlachthofbetreiber könnte auch eine Sonder-Prüfbox zur Verfügung haben, in der auffällige Tiere noch weitergehender überprüft werden. In dieser Sonderbox könnte eine dreidimensional bewegliche Arretierungsbox installiert sein, die rotiert und auch gekippt werden kann. Die Rinder könnte in dieser Box auch geröntgt werden oder mit speziellen neurophysiologischen Testungen apparativ über Anbringungen von Kopfkappen oder Vergurtungen, die Kontaktelektroden tragen noch genauer gecheckt werden. Alle Ergebnisse und äußeren Reaktionen und Verhaltensweisen werden synchron aufgezeichnet.

Generell können die Boxen oder Prüfumgebungen zentral gesteuert und überwacht werden. In der Überwachungszentrale kann speziell geschultes Personal oder auch ein Tierarzt sitzen. Dieses Personal kann dann sofort und mit Hilfe auch der automatischen Auswertungen der Computer eine Bewertung des Zustandes des Rindes durchführen oder ferngesteuert auf die apparativen Ausstattungen einwirken.

Diese beschriebenen, innovativen Prüfungen der Nutztiere können teils parallel durchgeführt werden, bzw. schnell hintereinander vollzogen werden, so dass der Zeitbedarf nur bei 3-5 Minuten liegen würde. Werden in einem großem Schlachthof mehrere Prüfboxen gleichzeitig genutzt, so können in kurzer Zeit viele Tiere ohne Beeinträchtigung des Betriebsablaufes überprüft werden.

Dem Schlachthofbetreiber steht somit erstmals ein hochwertiges Instrumentarium zur objektiven Prüfung des Gesundheits- und Krankheitszustandes seiner Tiere zur Verfügung, das große Tierzahlen bewältigt, den Betriebsfluss nicht stört und höchsten Anforderungen an die Überwachung und Qualitätssicherung entspricht.

## Patentansprüche

1. Verfahren zur Dokumentation des Gesundheits- und Krankheitszustandes großer Nutztiere, **dadurch gekennzeichnet, dass** jedes Nutztier in eine standardisierte, definierte Prüfumgebung eingebracht wird, in der rechnergesteuert, halb- oder vollautomatisiert und nicht invasiv die optisch-apparativ sichtbaren und die neurophysiologischen-neuromuskulären Zustandsbilder und Reaktions- und Verhaltensweisen des Tieres auf standardisierte, definierte, rechnergestützte akustische, optische, mechanische, taktile, neurophysiologische oder elektrische Provokationsreize aufgezeichnet und gespeichert werden.

2. Verfahren nach Anspruch 1, **dadurch gekennzeichnet, dass** die Zustandsbilder und Reaktions- und Verhaltensweisen analysiert und - vorzugsweise sofort - bewertet werden.

3. Verfahren nach Anspruch 1, **dadurch gekennzeichnet, dass** als Prüfumgebung festgelegte Prüfstrecken in Tierställen oder Schlachthäusern, festgelegte Prüfräume in Tierställen, Prüfboxen, Prüfzimmer oder überdachte Prüfunterstände bei Freilandhaltung genutzt werden.

4. Verfahren nach Anspruch 1 und 2, **dadurch gekennzeichnet, dass** Prüfboxen in speziellen Aufbauten von Lastkraftwagen zum mobilen und teilstationären Einsatz der Prüfboxen verwendet werden.

5. Verfahren nach Anspruch 1, **dadurch gekennzeichnet, daß** die Tierprüfungen durch Bewegungsmelder oder Kontakte automatisch eingeschaltet werden.

6. Verfahren nach Anspruch 1, **dadurch gekennzeichnet, daß** die Bewertungen und Daten der Prüfungen zu Tierärzten und Tierkliniken übertragen und von diesen spezielle Prüfprogramme angesteuert werden können.

7. Verfahren nach Anspruch 1, **dadurch gekennzeichnet, dass** die in den Prüfumgebungen eingesetzten Tierarretierungen und/oder in den Tierarretierungen Gestängeelemente bewegt, rotiert oder gekippt werden, um das Tier zu definierten Bewegungen zwingen oder taktil zu reizen.

8. Verfahren nach Anspruch 1, **dadurch gekennzeichnet, dass** zumindest Teilbereiche des Bodens der Prüfvorrichtung bewegt werden.

9. Verfahren nach einem der Ansprüche 1 bis 8, **dadurch gekennzeichnet, dass** mehrere Prüfumgebungen, insbesondere in Form von Prüfboxen, nebeneinander oder hintereinander in Form einer Prüfreihe oder Prüfkette genutzt werden und jede Prüfumgebung bzw. Prüfbox verschiedene Prüfschwerpunkte ausführt.

10. Verfahren nach Anspruch 1, **dadurch gekennzeichnet, dass** zusätzlich telethermographische Prüfungen, Woodlichtanalysen, Röntgen- und Ultraschalluntersuchungen, dreidimensionale Vermessungen der Tiere, Gewichts- und Größenmessungen oder Prüfungen der Gesichtsmimik bzw. der Augenbewegungen der Tiere erfolgen.

11. Verfahren nach Anspruch 1, **dadurch gekennzeichnet, dass** die Tiere im Verlaufe des Lebens mehrfach untersucht werden und deren Bewertung miteinander und mit den Bewertungen der jeweiligen Herde verglichen werden.

12. Vorrichtung zur Dokumentation des Gesundheits- und Krankheitszustandes großer Nutztiere, **dadurch gekennzeichnet, dass** eine Prüfvorrichtung vorgesehen ist, die Mittel zum Ausüben von akustischen, optischen, mechanischen, taktilen, neurophysiologischen oder elektrischen Provokationsreizen aufweist, und daß Mittel zum Aufzeichnen und Speichern der dadurch hervorgerufenen Zustandsbilder und Reaktions- und Verhaltensweisen des Tieres vorgesehen sind.

13. Vorrichtung nach Anspruch 12, **dadurch gekennzeichnet, dass** Mittel zum Analysieren und Bewerten der Zustandsbilder und Reaktions- und Verhaltensweisen des Tieres vorgesehen sind.

14. Vorrichtung nach Anspruch 12, **dadurch gekennzeichnet, dass** Mittel zum Bewegen, Rotieren oder Kippen der als Prüfumgebung eingesetzten Tierarretierungen und/oder Gestängeelementen in den Tierarretierungen vorgesehen sind.

15. Vorrichtung nach Anspruch 12, **dadurch gekennzeichnet, dass** zumindest Teilbereiche des Bodens der Prüfvorrichtung bewegbar sind.

16. Vorrichtung nach Anspruch 12, **dadurch gekennzeichnet, dass** zur neurophysiologischen und neuromuskulären Prüfung spezielle Elektroden in Bürsten oder Krallenformen mit Bandhalterungen oder an dreidimensional steuerbaren Kontaktarmen oder mit Elektrodenvergurtungen für den Kopfsitz vorgesehen sind.

17. Vorrichtung nach Anspruch 12, **dadurch gekennzeichnet, dass** zusätzlich Mittel für telethermographische Prüfungen, Woodlichtanalysen, Röntgen- und Ultraschalluntersuchungen, dreidimensionale Vermessungen der Tiere, Gewichts- und Größenmessungen oder Prüfungen der Gesichtsmimik bzw. der Augenbewegungen der Tiere vorgesehen sind.

18. Vorrichtung nach Anspruch 12, **dadurch gekennzeichnet, dass** mindestens eine Prüfvorrichtung auf einer Fördervorrichtung für den Transport der Nutztiere angeordnet ist.

19. Vorrichtung nach Anspruch 12, **dadurch gekennzeichnet, dass** mehrere Prüfvorrichtungen sternförmig angeordnet sind.

20. Vorrichtung nach Anspruch 12, **dadurch gekennzeichnet, dass** die Prüfvorrichtung transportierbar ist.
